Europäisches Patentamt

European Patent Office

Office européen des brevets

(11) Publication number: **0 267 005 B1**

# EUROPEAN PATENT SPECIFICATION

(45) Date of publication of patent specification: **23.12.92**  (51) Int. Cl.⁵: **A61K 37/02**, A61K 47/00, A61K 47/48

(21) Application number: **87309722.4**

(22) Date of filing: **03.11.87**

(54) Compositions for preventing secondary cataracts.

(30) Priority: **04.11.86 US 927318**

(43) Date of publication of application:
**11.05.88 Bulletin 88/19**

(45) Publication of the grant of the patent:
**23.12.92 Bulletin 92/52**

(84) Designated Contracting States:
**AT BE CH DE ES FR GB GR IT LI LU NL SE**

(56) References cited:
**EP-A- 0 044 167**
**EP-A- 0 140 109**
**EP-A- 0 173 648**
**US-A- 4 590 071**

(73) Proprietor: **BAYLOR COLLEGE OF MEDICINE**
**One Baylor Plaza**
**Houston, TX 77030(US)**

(72) Inventor: **Lam, Dominic Man-Kit**
**9 Wedgewood Glen**
**The Woodlands, TX 77380(US)**

(74) Representative: **Cresswell, Thomas Anthony et al**
**J.A. Kemp & Co. 14 South Square Gray's Inn**
**London WC1R 5EU(GB)**

## Description

The field concerns methods and compositions for inhibiting secondary cataracts using specific receptor cytotoxic compositions.

Extracapsular cataract extraction is a desirable method for removing cataracts due to a lower incidence of post-operative complications in terms of cystoid macular edema and possible retinal detachment with this technique. The availability of an improved extracapsular extraction technique such as phacoemulsification and the requirement of an intact posterior lens capsule for implantation of a wide variety of intraocular lenses has further supported extracapsular cataract extraction use. However, this surgical method is accompanied by a significant incidence of posterior lens capsule opacification, which may require additional surgical procedures (posterior capsulotomy or repolishing of the posterior lens capsule) to obtain good vision. The pathogenesis of posterior lens capsule opacification after extracapsular cataract extraction is reported to be due to proliferation of remnant lens epithelial cells on the posterior lens capsule to form abortive lens "fibers" and "bladder" cells (i.e., Elschnig's pearls).

Various techniques have been reported to inhibit this secondary cataract formation or posterior lens capsule opacification. Roy et al., Contact and Intraocular Lens Medical Journal (1979) 5:175-178 reported the use of vincristine and vinblastine. Radiation was also tried, which was reported to be promising. Methotrexate and retinoic acid have been reported for instillation in the anterior chamber of the eye to prevent posterior lens capsule opacification.

Production of monoclonal antibodies has been described. See, for example, Monoclonal Antibodies, eds. Roger H. Kennett, Thomas J. McKearn, Kathleen B. Bechtol, Plenum Press, New York, 1980; Nature - (1975) 256:495-497; U.S. Patent Nos. 4,271,145; 4,196,265; 4,172,124; 4,195,125; 4,262,090; and 4,294,927. See also, U.S. Patent No. 4,432,751, which discloses the combination of monoclonal antibodies and complement for preventing secondary cataracts.

Compositions are provided for preventing secondary cataracts, especially by inhibiting posterior lens capsule opacification after extracapsular cataract extraction. These are for use in methods wherein cytotoxic conjugates capable of binding specifically to epithelial cells are introduced into the anterior chamber of the eye prior to, concurrently with or subsequent to the extracapsular cataract extraction. Of particular interest is introducing non-cytotoxic agents into the anterior chamber prior to the introduction of the cytotoxic conjugates, where the non-cytotoxic agents and cytotoxic conjugates have substantially the same binding affinity for epithelial cells. This is effective in preventing opacification of the posterior lens capsule. The use of the cytotoxic conjugates in the manufacture of a pharmaceutical composition for the prevention of secondary cataracts is also within the invention.

The method of use of the compositions comprises instilling into the anterior chamber of the eye cytotoxic conjugates specific for epithelial cells, so as to substantially inhibit the proliferation of the lens epithelial cells. The cytotoxic conjugates are substantially specific for the lens epithelial cells and have low or no cross-reactivity with other cells found in the anterior chamber, such as fibroblasts, melanocytes, corneal endothelial cells, etc., desirably also other epithelial cells, e.g., corneal epithelial cells. Preferably, prior to instillation of the cytotoxic conjugate and prior to the extracapsular cataract extraction, a non-cytotoxic agent is instilled into the anterior chamber, where the non-cytotoxic agent is cross-reactive with or has substantially the same binding specificity as the cytotoxic conjugate, so as to bind to any cells in the anterior chamber which are in contact with the anterior chamber having homologous determinant or antigenic sites.

The cytotoxic conjugate is a conjugate of a protein macromolecule capable of binding substantially specifically to epithelial cells, particularly lens epithelial cells, as compared to other cells which may be present in or in contact with the anterior chamber of the eye. For the most part, cytotoxic conjugates will be conjugates of a monoclonal antibody or its equivalent with a cytotoxic agent. The monoclonal antibody may be produced as a result of hybridoma formation and expression by the hybridoma, whether in culture or present as ascites, a monoclonal antibody fragment, such as Fab, F(ab')$_2$, Fv, a recombinant variable region or a T-cell receptor. The monoclonal antibodies and receptors may be any mammalian species, including murine, rabbit or human, or combinations thereof, such as chimeric antibodies, having a human constant region and a mouse or other mammalian source variable region. The antibodies may be any class or subclass, such as IgA, IgD, IgG, IgM, and may include IgG1, 2a, 2b, or 3 or the human equivalent thereof.

The methods for preparing the monoclonal antibodies are well established as evidenced by the numerous references described above. Furthermore, the resulting monoclonal antibodies may be isolated and modified by truncating the constant region by various peptidase digestions. The monoclonal antibodies may be reduced to provide for Fab fragments with available mercaptan sites for conjugation to other compositions. T-cell receptors may be obtained as described in WO85/03947.

Various epithelial cells may be used as the immunogen, particularly lens epithelial cells, more particularly human epithelial cells, although other species may find use, e.g., primates. Whole cells are preferred, however homogenates, membrane fragments or the like, can be used.

The binding compositions having specificity for the epithelial cells, may be joined to a wide variety of toxic agents, microorganism or plant. Of particular interest are the toxic subunits of naturally occurring toxins, such as ricin, abrin, diphtheria toxin, etc. See for example Oeltmann and Heath, J. Biol. Chem. - (1979) 254:1022-1027; Yule and Neville Jr., Proc. Natl. Acad. Sci. USA (1980) 77:5483-5486; Gilliland et al., Proc. Natl. Acad. Sci. USA (1978) 75:5319-5323; U.S. Patent No. 4,379,145; GB2034324 and Masuho et al., Biochem. Biophys. Res. Crnn. (1979) 90:320-326; and Blythman, Nature (1981) 290:145.

Illustrative toxin A-chains or similarly effective moieties include diphtheria toxin A-chains, enzymically active proteolytic fragments from Pseudomonas aeruginosa exotoxin-A, ricin toxin A-chain, abrin A-chain, modeccin A-chain, and proteins found in various plants having similar activity, such as the plants e.g., Gelonium multiflorum, Phytolacca americana, Croton tiglium, Jatropha curcas, Momordic charantia, and wheat germ. Also, mutant species of the toxins of the species may be used, such as CRM45 (Boquet et al., Proc. Natl. Acad. Sci. USA (1976) 73:4449-4453).

The toxic agents and receptor may be linked, usually by a bond which is cleavable cytoplasmically. Convenient linkages include disulfide, particularly where the toxic agent has an intrinsic sulfur, or other links, such as peptide links, urea links, thioethers, imines, amides, imides or amidines. Functional groups which may find employment include carboxylic acid groups, amino groups, imines, aldehydes, isocyanates, mercaptans or olefins. In addition, more complex linking groups can be employed, where a group may be bound to one of the moieties in the conjugate to provide for convenient linkage to an intrinsic group of the other moiety. For example, the N-hydroxysuccinimide ester of m-maleimidoylbenzoic acid may be employed to prepare an amide of the toxin, which may then be linked through an available sulfur atom on the monoclonal antibody to provide a thioether.

Exemplary cytoxic conjugates may have the following formula:

$$(AS_n)\text{-}(S\text{-}X\text{-}R)_m$$

wherein:

$AS_n$ indicates the toxic agent having one or more sulfur groups as part of the agent; n is 1 to the number of sulfur groups present in the toxic agent which are present as available mercaptide groups, generally being up to about 4; R is a monoclonal antibody receptor or derivative thereof; and m is 1 up to n, usually being from 1 to 2; and X is a linking group and may be a bond or a group of from about 1 to 20, usually 1 to 12 atoms other than hydrogen, which include carbon, nitrogen, oxygen and sulfur. Sulfur will normally be bonded to carbon, particularly aliphatically saturated. X may be aliphatic, alicyclic, aromatic, heterocyclic or combinations thereof, generally having from 0 to 6, more usually from about 0 to 4, preferably about 1 to 4 heteroatoms, wherein oxygen and sulfur are present as oxo or non-oxo-carbonyl or the thio analogs thereof, or ether (including thioether), and nitrogen is present as amino or amido. For the most part the heteroatoms will bonded solely to carbon.

Illustrative groups linking the disulfide include aminoethylene 3-propanyl methylene carbonyl, α-succinimidyl, 3-propylenethiocarbonyl. The groups which may be used are for the most part conventional groups providing for a disulfide linkage. The disulfide compound is one which is capable of reacting with the cell-specific ligand, whereby a mercaptide group may be displaced from the disulfide, resulting in a new disulfide linkage between the toxic agent and the ligand.

For the most part, the linkages will be aliphatic of from 1 to 6 carbon atoms, providing for an amide bond to the receptor, although this is primarily a matter of convenience, not necessary to the operability of the subject compositions.

Other toxic agents may also be used, such as bismuth non-diffusively linked to the monoclonal antibodies or receptors as described by Waldman, J. Amer. Med. Assoc.

Alternatively, liposomes may be linked to the monoclonal antibodies or receptors, where the liposomes contain various cytotoxic agents, such as methotrexate, 5-fluorouracil, or any of the above toxins. See, for example, Szoka and Papahadjopoulos, Proc. Natl. Acad. Sci. USA (1978) 75:4194-4198 and Szoka et al., Biochem. et Biophys. Acta. (1980) 601:559-571 for the preparation of liposomes. Linking of antibodies to the liposome has been amply described in the literature, see for example Heath et al., Proc. Natl. Acad. Sci. USA (1983) 80:1377-1381 and Leserman et al., Nature (1981) 293:226-228.

Other cytotoxic agents conjugated to the receptor may also be employed in conjunction with the subject process of this invention. The conjugate will be sufficient to provide the cytotoxic effect without the addition of ancillary agents.

3

The preferred method of using subject compositions will involve initial incision about the cornea and instillation of about 25-200, preferably about 50-150, more preferably about 100 $\mu$l of a noncytotoxic agent capable of specifically binding to a site cross-reactive with the cytotoxic conjugate. For the most part, this agent will be a monoclonal antibody or a specific binding fragment thereof. Generally, the solution will be a physiologically acceptable solution, which may be saline, phosphate-buffered saline, or the like and will have a concentration of $10^8$-$10^{13}$, more usually $10^9$-$10^{12}$ antibodies/ml.

The antibodies or fragments thereof will bind to all sites which may be cross-reactive with the cytotoxic conjugate and will also bind non-specifically to "hot spots" which may be present within the anterior chamber of the eye. In this way, the cells will be protected from the cytotoxic agent.

The use of the cross-reactive binding agent will be of particular importance where the cytotoxic conjugate has cross-reactivity with cells other than lens epithelial cells. By use of the prior instillation of the crossreactive non-cytotoxic receptor, cytotoxic conjugates may be prepared which crossreact to varying degrees with cells, particularly epithelial cells, other than lens epithelial cells.

After instillation of the non-cytotoxic binding agent and incubation for a sufficient time for the binding agent to bind to its homologous antigen, the anterior chamber may be flushed to remove the contents of the anterior chamber.

Either before but usually after extracapsular cataract extraction, the cytotoxic conjugate will be introduced into the anterior chamber in a physiologically acceptable medium (see above) in an amount of from 25-200, more usually from 50-150 $\mu$l of the cytotoxic conjugate, which conjugate will be present in an amount sufficient to substantially completely or completely kill all of the lens epithelial cells. The number of cytotoxic molecules will usually be in the range of $10^7$-$10^{11}$ molecules/ml. Generally, the cytotoxic effect will be realized within a relatively short time after the instillation of the cytotoxic conjugates, usually in about 0.5 hour, or shortly thereafter, when inhibition of protein synthesis is used to evaluate onset of the cytotoxic effect. However, if viability is assessed by some other mechanism, e.g., vital stains, etc., It may be hours to days before a cytotoxic effect (e.g., cell death) is noted.

The subject compositions can be provided as kits for use in one or more operations. The kits will include the non-cytotoxic agent and the cytotoxic conjugate, either as concentrates, which may be further diluted prior to use or at the concentration of use, where the vials may include one or more dosages. Conveniently, single dosages may be provided in syringes, contained in sterilized containers, so that the physician may employ the syringes directly, where the syringes will have the desired amount and concentration of agents. Thus, the kit may have a plurality of syringes containing the cytotoxic conjugate as well as the non-cytotoxic agent in appropriate proportional amounts. Where the syringes contain the formulation for direct use, usually there will be no need for other reagents for use with the method.

The following examples are offered by way of illustration and not by way of limitation.

EXPERIMENTAL

Example 1

Preparation of Monoclonal Antibodies

A. Immunization

Mice (BALB/c) were immunized with human cervical carcinoma epithelial cells (ME180) by injecting i.p. about $5 \times 10^6$ cells/200$\mu$l three times over 2 week intervals and i.v. 3 days before isolation of the spleen with $5 \times 10^6$ cells/200$\mu$l. Additional mice (BALB/c) were immunized with human lens epithelial cells, obtained following cataract surgery, emulsified in Freunds Complete Adjuvant. About $5 \times 10^5$ cell equivalents per 200$\mu$l were given per animal for the primary injection. Additional injections consisting of equivalent cell material emulsified in Freunds incomplete were given i.m. 3 weeks apart. These mice received two additional injections of either ME180 cells or human amnion cells (WISH) prior to harvesting spleens for fusion.

B. Fusion

Spleens from immunized animals were removed three to five days following the last injection and prepared as a single cell suspension. The lymphocytes then were fused with P3-X-63/Ag8.653 mouse myeloma cells under conventional conditions. See U.S. Patent No. 4,432,751. Following fusion, the cells were resuspended in Iscoves medium containing hypoxanthine, aminopterin, and thymidine (HAT medium) and placed in wells according to the number of myeloma cells to give a density of about $10^4$ cells/well. The cells were fed on days 5, 12, and 15 by removing and replacing half of the medium. Cultures identified as positive for antibody secretion by screening assays, were tranfered to 24 well plates containing 1ml Iscoves medium containing hypoxanthine and thymidine (HT).

C. Screening

(i) ME180 immunization: For screening, the hybridoma supernatant ($100\mu l$) and $100\mu l$ ME180 cells ($2x10^5$ cells/ml) were incubated at 4° for one hr. and then at 37° overnight in 96 well microtiter plates. The wells were viewed microscopically to detect disturbances (disturbed adhesion). Cells showing signs of disturbance were isolated and cloned 3 times. One of the hybridomas testing positively was designated 3D4. 3D4 produces an $I_gG_1$ monoclonal antibody as determined be gel diffusion using class specific antiserum (ICN).

(ii) HLE/ME180 immunization: For screening, hybridoma supernatants were tested for binding to ME180 cells by ELISA. Bound mouse immunoglobulin was detected by goat anti-mouse IgG-horse radish peroxidase. One of the hybridomas testing positively was designated 4757. This hybridoma produces an $IgG_1$, as determined by gel diffusion.

(iii) HLE/WISH immunization: For screening, hybridoma supernatant was tested for binding to WISH cells by ELISA. Bound mouse immunoglobulin was detected as described. One of the hybridomas testing positively was designated 4197. This hybridoma produces an $IgG_{2a}$ antibody as determined by gel diffusion.

(iv) Human lens epithelial cells antigen: Hybridoma supernatants from fusions performed with spleen cells obtained from human lens epithelial cell immunized mice were tested by ELISA. Ninety-six well plates were coated with a Nonidet P-40 soluble fraction of human lens epithelial cells obtained following cataract surgery. Fifty microlitres per well of this soluble fraction were added to 96 well plates, dried and fixed with 0.05% glutaraldehyde for 15 minutes at 25°C. The plates were washed and incubated with cell culture medium containing 10% FBS for 60 minutes at room temperature. (Kelleher, et al., Cancer Immunol Immunother (1983) 14:185-190 and Mujoo et al., J. Biol. Chem. (1986) 261:10299-10305). Hybridoma supernatants, $50\mu l$/well, were added to the wells and incubated for 60 minutes. The wells were then washed and bound antibody detected by goat anti-mouse IgG-horse radish peroxidase. Cultures testing positive were expanded to 24 well plates and tested further for cellular binding specificity.

D. Cell Binding of Monoclonal Antibody

Supernatants from hybridomas 3D4, 4197 and 4757 were tested by ELISA for ability to bind to both normal and tumor cells. Adherent cell lines were grown to confluence in 96 well plates and then fixed with 0.05% glutaraldehyde for 10 minutes. Suspension cultures were attached to poly L-lysine coated plates and fixed as above. Monoclonal antibody in culture medium was added to the wells and incubated at 37°C for 1 hour. The plates were washed three time and bound mouse IgG detected with goat anti-mouse IgG conjugated to horse radish peroxidase. Binding of the monoclonal antibodies to various cell types is shown in Table 1.

### Table 1

### Binding of 3D4, 4197 and 4757 Antibodies to
### Various Cell Types

| Cell line | Tissue | Cell Type | Absorbance* at 450 nm | | |
|---|---|---|---|---|---|
| | | | 3D4 | 4197 | 4757 |
| ME180 | human cervix | epithelial | 0.40 | 0.17 | 0.28 |
| WISH | human amnion | epithelial | 0.21 | 0.52 | 0.16 |
| COLO 320 | colon | adenocarcinoma | 0.01 | 0.00 | 0.00 |
| MRC5 | human skin | fibroblast | 0.14 | 0.06 | 0.03 |
| Daudi | Burkitt lymphoid | lymphoma | 0.01 | 0.07 | 0.02 |
| Y79 | retinoblastoma | | 0.01 | 0.06 | 0.00 |
| RPMI 7932 | melanoma | melanoma | 0.03 | 0.00 | 0.00 |
| RLE | rabbit lens | epithelial | 0.70 | 0.00 | 0.43 |

* Absorbance at 450 nm: Increasing absorbance reflects increased binding of antibody to cells.

The antibodies prepared using human cervical carcinoma cells or other epithelial cells as an immunogen are capable of binding to rabbit lens epithelial cells, as well as epithelial cell lines derived from a variety of different tissues. There is little or no binding to cells of non-epithelial origin. In addition, the antibodies from 3D4, 4197 and 4757 have been shown to bind human lens epithelial cells by immunocytochemical staining. These results are shown in Table 2.

### Table 2

### Binding to HLE cells*

| | Antibody | | |
|---|---|---|---|
| | 3D4 | 4197 | 4757 |
| Relative staining | ++ | +++ | ++ |

* Media control, or in relevant antibodies did not stain human lens epithelial cells.

Concomittant addition of $^{125}$I-3D4 and excess unlabeled 3D4 to the cultures inhibited the binding of the labeled 3D4 by 90%. These results are shown in Table 3.

## Table 3
## Specificity of Binding of $^{125}I$-3D4 to Epithelial Cells

| | CPM $^{125}I$ | |
| --- | --- | --- |
| Addition | RLE Cells | ME180 Cells |
| $^{125}I$-3D4 in buffer control | 4480 | 2470 |
| $^{125}I$-3D4 in excess 3D4IgG | 460 | 400 |
| % Inhibition | 90% | 84% |

Example 2

Preparation of Toxin-A Chain-Antibody Conjugates

A. Preparation Toxin A-Chain

(i) Diptheria Toxin: Fragment A (DTA) is prepared from diptheria toxin (Connaught Laboratories) as described (Chung and Collier, Biochem. Biophys. Acta (1977) 483:248-257) and is heated to 80°C for 10 minutes to inactivate any residual traces of toxin. Enzymic activity to DTA is assayed by ADP-ribosylation of wheat germ elongation factor 2 with $^{14}C$-NAD as substrate.

(ii) Ricin Toxin: Ricin toxin was purified from castor beans (A. H. Hummert Seed Co., St. Louis, Missouri) by affinity chromatography on Sepharose® 4B. (Nicolson and Blaustein, ibid. (1972) 266:543-54 and modified by Cawley et al., Arch. Biochem. Biophys. ( ) 190:744-755). Ricin toxin A-chain (RTA) was purified from whole ricin toxin by reduction from 2-mercaptoethanol and chromatography on Cellex-D (Bio-Rad) (Olsnes and Pihl, Biochem. (1973) 12:3121-3126). RTA is freed of residual traces of ricin toxin by repeated cycling on Sepharose 4B columns.

B. Synthesis of Toxin-SS Antibody Conjugates

(i) Synthesis of (DTA)-SS antibody conjugates: Antibody 3D4 (7.0ml, 2mg/ml) is dialyzed against Dulbecco's phosphate buffered saline (DPBS Gibco), pH7.4, containing antibiotic-antimycotic solution (Gibco, 5.0ml per liter). N-succinimidyl-3-(2-pyridyldithio) propionate (SPDP) (0.186ml, 10mM) in absolute ethanol is added to the antibody with vigorous mixing. The mixture is allowed to react for 30 minutes at room temperature and then dialyzed against two 1 liter changes of the same buffer. After dialysis the antibody preparation is analyzed for 2-pyridyldisulfide content as described (Stuchbury et al., Biochem. J. (1975) 151:417-432). DTA (3.0ml, 2.5mg/ml) is reduced by addition of 0.3ml of 1.0M dithiothreitol, pH7.0, for 30min at room temperature and desalted on Sephadex® G-25 (2.6 X 12 cm column) equilibrated with the buffer described above. Peak fractions from the column are pooled (11.0ml, 0.53mg/ml) and mixed with PDP-(3D4) antibody (7.0ml, 2.1mg/ml). Final concentration of DTA and antibody are $1.5 \times 10^{-5}M$ and $5.5 \times 10^{-6}M$, respectively. The final molar ratio of DTA to antibody in the reaction mixture is about 3. The crude conjugate preparation (18.0ml) is concentrated to a final volume of 0.9ml by ultracentrifugation on an Amicon YM-10 membrane. Crude (DTA)-SS-(3D4) (9.0ml) is chromatographed on a Sephacryl® S-2000 column (2.6 X 106cm, 22.1ml/h flow rate) equilibrated with DPBS buffer. Each fraction (6.2ml) is analyzed for ADP-ribosylation activity and by sodium dodecylsulfate/polyacrylamide gel electrophoresis (SDS/PAGE). Fractions 30-40 are pooled, concentrated on an Amicon YM-10 membrane, and are used in cytotoxicity assays after filter sterilization.

(ii) Synthesis of (RTA)-SS-(3D4) antibody conjugate: (RTA)-SS(3D4) was synthesized as described by Kernan et al. J. Biol. Chem. (1984) 133:137-146. Briefly, 3D4 (1 to 2 mg/ml) was dialyzed against 0.1 M NaPO$_4$, 0.1 M NaCl, pH 7.7, and 15- to 20-fold molar excess of n-succinimidyl-3-(2 pyridyldithiopropionate (SPDP) was added to the antibody with vigorous mixing. After incubation at room temperature for 30 min. the pyridyldithiopropionate (PDP)-modified antibody solution was dialyzed against two changes of PBS. After dialysis, the PDP group to antibody ratio was determined. RTA was initially reduced by the addition of dithiothreitol at a final concentration of 50 mM, followed by incubation for 1 hour at room temperature. The RTA was then dialyzed extensively against PBS (4°C) to remove any residual reducing agent. The RTA was then concentrated by using an Amicon stirred cell fitted with a YM10 membrane to a final concentration of 4 mg/ml. Five- or 10-fold molar excess RTA was then added to the PDP-antibody solution and incubated for 16 hr at 4°C. The RTA-3D4 conjugate was purified by chromatography on Sephacryl® S-200.

C. Effect of 3D4-Ricin A Conjugates on Target Cells:

(i) Cytotoxicity: Target cells (ME180, RPMI 7932; rabbit lens epithelial cells (RLE)) were plated in 96-well plates to achieve 25% confluence. Ricin A-conjugate (stock solution 1 mg protein/ml) or control media was added at the indicated dilutions and incubated for 10 min at either 37° or 25°C. The supernatant was removed, the cells washed twice and fresh medium without toxin conjugate added to the wells. The plates were incubated at 37°C until control wells were confluent. Cell density then was determined by conversion of the yellow dye 3-(4,5-dimethylthiazol-2-yl)-2,5-diphenyl tetrazolium bromide (MTT) to a purple product by living cells in direct proportion to cell number and metabolic activity. Appearance of the product was measured spectrophotometrically. Mosmann, J. Immunol. Methods (1983) 65:55. Percent reduction in cells was calculated by:

$$\% \text{ reduction in cells} = 100 - \frac{\text{Cell density (absorbance) in test}}{\text{Cell density (absorbance) in control}} \times 100$$

The results are shown in Table 4, below.

Table 4

Cytotoxicity of 3D4-ricin A for Cells:

10 minute incubation at 37° and 25°C

Percent Reduction in Cells

| 3D4-ricin A Conjugate Dilution | Cell Type | | | | | |
|---|---|---|---|---|---|---|
| | ME180 | | RPMI 7932 | | RLE | |
| | 37°C | 25°C | 37°C | 25°C | 37°C | 25°C |
| 1:10 | 93 | 88 | 24 | 0 | 84 | NT* |
| 1:20 | 96 | 88 | 18 | 0 | 85 | |
| 1:40 | 95 | 88 | 6 | 0 | 84 | |
| 1:80 | 91 | 87 | 15 | 11 | 85 | |
| 1:160 | 81 | 74 | 9 | 11 | 83 | |
| 1:320 | 53 | 53 | 0 | 4 | 84 | |
| 1:640 | 39 | 42 | 0 | 0 | 80 | |
| 1:1280 | 24 | 10 | 2 | 0 | 70 | |
| 1:2560 | 11 | 8 | 5 | 0 | 60 | |
| 1:5120 | 1 | 0 | 15 | 0 | 59 | |

\* NT - Not tested

Proliferation of specific target epithelial cells was significantly prevented by exposure to as little as 10 $\mu$g protein/ml of 3D4-ricin A conjugate, a concentration which had no effect on RPMI 7932 control cells.

(ii) Inhibition of Growth: Human lens epithelial cells obtained from cataract surgery were incubated in RPMI 1640 containing 10% FBS. For the experiment, the culture medium was removed and replaced with 3D4 RTA immunoconjugate in medium (20 $\mu$g/ml) or medium alone for 6 hours at 37°C. Following this incubation, cells were washed and given fresh culture media without conjugate and incubated for 48 hours. $^3$H-leucine was added to all cultures 24 hours prior to harvest of TCA precipitable protein. Thorpe et al., Eur. J. Biochem (1981) 116:447; Domingo and Trowbridge Methods in Enzymology (1985) 112:238; Vitetta et al. Proc. Nat. Acad. Sci.USA (1983) 80:6332. The results are shown in Table 5.

## Table 5

### Inhibition of $^3$H-leucine Incorporation by
### Human Lens Epithelial Cells by 3D4-ricin A

| Culture Addition | CPM Incorporated |
| --- | --- |
| 3D4-Ricin A | 3,655 |
| Medium Alone | 22,255 |

Based on these data, it appears that the monoclonal antibody-toxin conjugate is being translocated into the lens epithelial cell where the cytotoxic portion is active.

Example 3

Inhibition of Cytoxic Effect of Conjugate by Prior Incubation With Unconjugated Monoclonal Antibody

To demonstrate protection of crossreactive cells which bind the conjugate specifically, unconjugated 3D4 or medium alone was added to the cultures 10 min. prior to 3D4-ricin toxin A conjugate addition. A dilution of conjugate (as indicated in Table 6) was then added to the cells, incubated for 60 min. at 37°C. Cells were given fresh medium and incubated for 3 days at 37°C. Cell density was determined with MTT as described above (See Example 2).

## Table 6
### Effect of Preincubation of Cells
### With Unconjugated Monoclonal Antibody*

| | Treatment | |
|---|---|---|
| 3D4 RTA (X 100) | 3D4 RTA | 3D4 IgG + 3D4 RTA[1] |
| 2 | 10 | 67 |
| 4 | 21 | 95 |
| 8 | 20 | 99 |
| 16 | 32 | 98 |
| 32 | 50 | 98 |
| 64 | 60 | 98 |
| 128 | 91 | 98 |
| 256 | 87 | 98 |
| 512 | 100 | 100 |

* Expressed as a percentage of control cells not treated with conjugate.

[1] Cells were exposed to 3D4 IgG at 100 µg/ml for 10 minutes. Cells were washed 3 times and 3D4 RTA added.

Example 4

Effect of Conjugate on Proliferation of Lens Epithelial Cells in Vivo

Female New Zealand White rabbits, 3kg, underwent extracapsular lens removal under general anesthesia. (Emery et al., The C.V. Mosby Company, (1983).) Prior to surgery, animals received an injection of 3D4 IgG in balanced salt solution (BSS) (Alcon Labs, Fort Worth, TX) into the anterior chamber. Anterior capsulotomy was performed and the lens extracted with a Kelman phacoemulsifier (Cooper Vision, Irvine, CA). Lens material was aspirated/irrigated from the eye with BSS. Just before closure, 200 $\mu$l of immunoconjugate or BSS was injected into the anterior chamber. Eyes were treated externally with broad spectrum antibiotic ointment and steroids. Eyes were examined for evidence of remnant lens epithelial cell proliferation.

## Table 7
### Inhibition of Lens Epithelial Cell
### Proliferation Following Treatment
### With 3D4-RTA Conjugate

| Treatment | Concentration Conjugate | Number Animals with Epithelial Cell Proliferation | Number of Animals Treated |
|---|---|---|---|
| BSS | -- | 6 | 6 |
| 3D4-RTA | 30µg/ml | 4 | 6 |
| 3D4-RTA | 100µg/ml | 3 | 8 |

By instilling the subject cytotoxic agents, particularly after instillation of the binding agent, remnant lens epithelial cells can be prevented from proliferating, thus avoiding secondary cataracts. The subject methods and compositions provide a safe and simple procedure for preventing secondary cataracts without injury to other tissue in the eye, thus providing a safe alternative to prior cytotoxic agents which have found use and are found to have general cytotoxic effects.

All publications and patent applications mentioned in this specification are indicative of the level of skill of those skilled in the art to which this invention pertains.

The invention now being fully described, it will be apparent to one of ordinary skill in the art that many changes and modifications can be made thereto.

## Claims

1. The use of a protein macromolecule capable of binding specifically to epithelial cells conjugated to a cytotoxic agent for the manufacture of a pharmaceutical composition for the prevention of secondary cataracts.

2. The use according to claim 1 wherein the protein macromolecule is an antibody or a fragment thereof.

3. The use according to claim 2 wherein the antibody is a monoclonal antibody.

4. The use according to any one of claims 1 to 3 wherein the cytotoxic agent is a microorganism or plant toxin A chain.

5. The use according to claim 4 wherein the A chain is the ricin A chain, the abrin A chain or the diphtheria toxin A chain.

6. The use according to any one of claims 1 to 5 wherein the pharmaceutical composition also comprises a non-cytotoxic agent having the same binding specificity as the conjugated protein macromolecule.

7. The use according to claim 6 wherein the non-cytotoxic agent is a monoclonal antibody or a fragment thereof.

8. The use according to any one of the preceding claims wherein the protein macromolecule is capable of binding specifically to lens epithelial cells.

9. A composition comprising
   (1) a protein macromolecule capable of binding specifically to epithelial cells conjugated to a cytotoxic agent and
   (2) a non-cytotoxic agent having the same binding specificity as the conjugated protein macro-

EP 0 267 005 B1

molecule

for use in the prevention of secondary cataracts wherein the non-cytotoxic agent (2) is for instillation prior to instillation of the conjugated protein macromolecule (1) in the same eye.

10. A composition according to claim 9 wherein the protein macromolecule is an antibody or a fragment thereof.

11. A composition according to claim 10 wherein the antibody is a monoclonal antibody.

12. A composition according to any one of claims 9 to 11 wherein the non-cytotoxic agent is a monoclonal antibody or a fragment of a monoclonal antibody.

13. A composition according to any one of claims 9 to 12 wherein the cytotoxic agent is a microorganism or plant toxin A chain.

14. A composition according to claim 13 wherein the A chain is the ricin A chain, the abrin A chain or the diphtheria toxin A chain.

15. A composition according to any one of claims 9 to 14 wherein the protein macromolecule is capable of binding specifically to lens epithelial cells.

16. A kit comprising a composition according to any one of claims 9 to 15 wherein the conjugated protein macromolecule is contained in a first container and the non-cytotoxic agent is contained in a second container.

17. An antibody or fragment thereof capable of binding specifically to lens epithelial cells conjugated to a cytotoxic agent, for use in treatment of secondary cataracts.

18. An antibody or fragment thereof according to claim 17 wherein the antibody or fragment thereof is specific for lens epithelial cells.

19. A conjugated antibody or fragment thereof according to claim 17 or claim 18 which is a monoclonal antibody.

20. A conjugated antibody or fragment thereof according to claim 17, claim 18 or claim 19 wherein the cytotoxic agent is a microorganism or plant toxin A chain.

21. A conjugated antibody or fragment thereof according to claim 20 wherein the A chain is a ricin A chain, an abrin A chain or a diphtheria toxin A chain.

22. A conjugated antibody or fragment thereof according to claim 20 wherein said plant toxin is Ricin.

**Patentansprüche**

1. Verwendung eines zur spezifischen Bindung an epitheliale Zellen fähigen Protein-Makromoleküls, das mit einem zytotoxischen Agens konjugiert ist, zur Herstellung einer pharmazeutischen Zusammensetzung zur Verhinderung von Nachstaren.

2. Verwendung nach Anspruch 1, worin das Protein-Makromolekül ein Antikörper oder ein Fragment davon ist.

3. Verwendung nach Anspruch 2, worin der Antikörper ein monoklonaler Antikörper ist.

4. Verwendung nach einem der Ansprüche 1 bis 3, worin das zytotoxische Agens eine Mikroorganismen- oder eine Pflanzentoxin A-Kette ist.

5. Verwendung nach Anspruch 4, worin die A-Kette die Ricin A-Kette, die Abrin A-Kette oder die Diphterietoxin A-Kette ist.

13

6. Verwendung nach einem der Ansprüche 1 bis 5, worin die pharmazeutische Zusammensetzung zusätzlich ein nichtzytotoxisches Agens enthält, das dieselbe Bindungsspezifität aufweist wie das konjugierte Protein-Makromolekül.

7. Verwendung nach Anspruch 6, worin das nicht-zytotoxische Agens ein monoklonaler Antikörper oder ein Fragment davon ist.

8. Verwendung nach einem der vorhergehenden Ansprüche, worin das Protein-Makromolekül dazu fähig ist, spezifisch an epitheliale Zellen der Linse zu binden.

9. Zusammensetzung, enthaltend
(1) ein zur spezifischen Bindung an epitheliale Zellen fähiges Protein-Makromolekül, das mit einem zytotoxischen Agens konjugiert ist und
(2) ein nicht-zytotoxisches Agens, das dieselbe Bindungsspezifität wie das konjugierte Protein-Makromolekül aufweist,
zur Verwendung bei der Verhinderung von Nachstaren, wobei das nicht-zytotoxische Agens (2) einzuträufeln ist, bevor das konjugierte Protein-Makromolekül (1) in dasselbe Auge eingeträufelt wird.

10. Zusammensetzung nach Anspruch 9, worin das Protein-Makromolekül ein Antikörper oder ein Fragment davon ist.

11. Zusammensetzung nach Anspruch 10, worin der Antikörper ein monoklonaler Antikörper ist.

12. Zusammensetzung nach einem der Ansprüche 9 bis 11, worin das nicht-zytotoxische Agens ein monoklonaler Antikörper oder ein Fragment eines monoklonalen Antikörpers ist.

13. Zusammensetzung nach einem der Ansprüche 9 bis 12, worin das zytotoxische Agens eine Mikroorganismen- oder eine Pflanzentoxin A-Kette ist.

14. Zusammensetzung nach Anspruch 13, worin die A-Kette die Ricin A-Kette, die Abrin A-Kette oder die Diphterietoxin A-Kette ist.

15. Zusammensetzung nach einem der Ansprüche 9 bis 14, worin das Protein-Makromolekül zur spezifischen Bindung an epitheliale Zellen der Linse fähig ist.

16. Kit, umfassend eine Zusammensetzung nach einem der Ansprüche 9 bis 15, worin das konjugierte Protein-Makromolekül in einem ersten Behälter und das nicht-zytotoxische Agens in einem zweiten Behälter enthalten ist.

17. Zur spezifischen Bindung an epitheliale Zellen der Linse fähiger Antikörper oder ein Fragment davon, der/das mit einem zytotoxischen Agens konjugiert ist, zur Verwendung bei der Behandlung von Nachstaren.

18. Antikörper oder Fragment davon nach Anspruch 17, worin der Antikörper oder das Fragment davon für epitheliale Zellen der Linse spezifisch ist.

19. Konjugierter Antikörper oder Fragment davon nach Anspruch 17 oder Anspruch 18, dadurch gekennzeichnet, daß er ein monoklonaler Antikörper ist.

20. Konjugierter Antikörper oder Fragment davon nach Anspruch 17, Anspruch 18 oder Anspruch 19, worin das zytotoxische Agens eine Mikroorganismen- oder eine Pflanzentoxin A-Kette ist.

21. Konjugierter Antikörper oder Fragment davon nach Anspruch 20, worin die A-Kette eine Ricin A-Kette, eine Abrin A-Kette oder eine Diphterietoxin A-Kette ist.

22. Konjugierter Antikörper oder Fragment davon nach Anspruch 20, worin das Pflanzentoxin Ricin ist.

**Revendications**

**EP 0 267 005 B1**

1. Utilisation d'une macromolécule de protéine apte à se lier spécifiquement à des cellules épithéliales conjuguée à un agent cytotoxique pour la fabrication d'une composition pharmaceutique pour la prévention de cataractes secondaires.

2. Utilisation selon la revendication 1, dans laquelle la macromolécule de protéine est un anticorps ou un fragment de celui-ci.

3. Utilisation selon la revendication 2, dans laquelle l'anticorps est un anticorps monoclonal.

4. Utilisation selon l'une quelconque des revendications 1 à 3, dans laquelle l'agent cytotoxique est un microorganisme ou la chaîne A d'une toxine végétale.

5. Utilisation selon la revendication 4, dans laquelle la chaîne A est la chaîne A de ricin, la chaîne A d'abrine ou la chaîne A de la toxine diphtérique.

6. Utilisation selon l'une quelconque des revendications 1 à 5, dans laquelle la composition pharmaceutique comporte également un agent non-cytotoxique présentant la même spécificité de liaison que la macromolécule de protéine conjuguée.

7. Utilisation selon la revendication 6, dans laquelle l'agent non-cytotoxique est un anticorps monoclonal ou un fragment de celui-ci.

8. Utilisation selon l'une quelconque des revendications précédentes, dans laquelle la macromolécule de protéine est apte à se lier spécifiquement à des cellules épithéliales du cristallin.

9. Composition comportant
   (1) une macromolécule de protéine apte à se lier spécifiquement à des cellules épithéliales conjuguée à un agent cytotoxique et
   (2) un agent non-cytotoxique présentant la même spécificité de liaison que la macromolécule de protéine conjuguée
   pour une utilisation dans la prévention de cataractes secondaires, dans laquelle l'agent non-cytotoxique (2) est destiné à être instillé avant l'installation de la macromolécule de protéine conjuguée (1) dans le même oeil.

10. Composition selon la revendication 9, dans laquelle la macromolécule de protéine est un anticorps ou un fragment de celui-ci.

11. Composition selon la revendication 10, dans laquelle l'anticorps est un anticorps monoclonal.

12. Composition selon l'une quelconque des revendications 9 à 11, dans laquelle l'agent non-cytotoxique est un anticorps monoclonal ou un fragment d'un anticorps monoclonal.

13. Composition selon l'une quelconque des revendications 9 à 12, dans laquelle l'agent cytotoxique est un microorganisme ou la chaîne A d'une toxine végétale.

14. Composition selon la revendication 13, dans laquelle la chaîne A est la chaîne A de ricin, la chaîne A d'abrine ou la chaîne A de la toxine diphtérique.

15. Composition selon l'une quelconque des revendications 9 à 14, dans laquelle la macromolécule de protéine est apte à se lier spécifiquement à des cellules épithéliales du cristallin.

16. Coffret comportant une composition selon l'une quelconque des revendications 9 à 15, dans lequel la macromolécule de protéine conjuguée est contenue dans un premier récipient et l'agent non-cytotoxique est contenu dans un second récipient.

17. Anticorps ou fragment de celui-ci apte à se lier spécifiquement à des cellules épithéliales du cristallin conjugué à un agent cytotoxique, pour une utilisation dans le traitement de cataractes secondaires.

15

18. Anticorps ou fragment de celui-ci selon la revendication 17, dans lequel l'anticorps ou un fragment de celui-ci est spécifique pour des cellules épithéliales du cristallin.

19. Anticorps conjugué ou fragment de celui-ci selon la revendication 17 ou la revendication 18 qui est un anticorps monoclonal.

20. Anticorps conjugué ou fragment de celui-ci selon la revendication 17, la revendication 18 ou la revendication 19 , dans lequel l'agent cytotoxique est un microorganisme ou la chaîne A d'une toxine végétale.

21. Anticorps conjugué ou fragment de celui-ci selon la revendication 20, dans lequel la chaîne A est une chaîne A de ricin, une chaîne A d'abrine ou une chaîne A de la toxine diphtérique.

22. Anticorps conjugué ou fragment de celui-ci selon la revendication 20, dans lequel ladite toxine végétale est le ricin.